# EUROPÄISCHE PATENTSCHRIFT

(11) **EP 1 070 132 B1**
(45) Veröffentlichungstag und Bekanntmachung des Hinweises auf die Patenterteilung: **23.09.2009**
(21) Anmeldenummer: 99915609.4
(22) Anmeldetag: 11.03.1999
(51) Int. Cl.: C12N 15/55, C12N 9/80, C12N 15/63, C12N 1/21, C12P 13/04, C12P 41/00

(54) **REKOMBINANTE L-N-CARBAMOYLASE AUS ARTHROBACTER AURESCENS, VERFAHREN ZUR HERSTELLUNG VON L-AMINOSÄUREN MITTELS DIESER**
RECOMBINANT L-N-CARBAMOYLASE DERIVED FROM ARTHROBACTER AURESCENS, AND A METHOD FOR PRODUCING L-AMINO ACIDS BY USING THE SAME
L-N-CARBAMOYLASE RECOMBINEE ISSUE D'ARTHROBACTER AURESCENS, PROCEDE DE PREPARATION DE L-AMINOACIDES AVEC LADITE L-N-CARBAMOYLASE RECOMBINEE

(30) Priorität: 02.04.1998 DE 19814813
(43) Veröffentlichungstag der Anmeldung: 24.01.2001
(73) Patentinhaber: Evonik Degussa GmbH, 45128 Essen (DE); Universität Stuttgart, 70569 Stuttgart (DE)
(72) Erfinder: ALTENBUCHNER, Josef, D-71154 Nufringen (DE); MATTES, Ralf, D-70619 Stuttgart (DE); PIETZSCH, Markus, D-70186 Stuttgart (DE); SYLDATK, Christoph, D-70186 Stuttgart (DE); WIESE, Anja, D-85386 Eching (DE); WILMS, Burkard, D-70563 Stuttgart (DE)
(74) Vertreter: Retzow, Stefan
(86) Internationale Anmeldenummer: PCT/EP1999/001681
(87) Internationale Veröffentlichungsnummer: WO 1999/051722

(56) Entgegenhaltungen:
- EP-A- 0 625 571
- FR-A- 2 728 905
- WILMS, B. ET AL.: "Cloning, nucleotide sequence and expression of a new L-N-carbamoylase gene from Arthrobacter aurescens DSM 3747 in E.coli" JOURNAL OF BIOTECHNOLOGY, Bd. 68, 19. Februar 1999 (1999-02-19), Seiten 101-113, XP004164275
- SIEMANN, M. ET AL.: "Detection and comparison of strains with selective L-hydantoin cleaving activity using polyclonal antibodies" BIOTECHNOLOGY TECHNIQUES, Bd. 7, Nr. 5, Mai 1993 (1993-05), Seiten 361-366, XP002125059
- GROSS, C. ET AL.: "Screening method for microorganisms producing L-aminoacids from D,L-5-monosubstituted hydantoins" BIOTECHNOLOGY TECHNIQUES, Bd. 1, Nr. 2, 1987, Seiten 85-90, XP002125060

## Beschreibung

Die vorliegende Erfindung richtet sich auf eine neue rekombinante (rec-) L-N-Carbamoylase aus Arthrobacter aurescens sowie auf ein Verfahren zur Herstellung von L-Aminosäuren mittels dieser Carbamoylase.

Die durch die neue rec-Carbamoylase vorteilhaft herstellbaren L-Aminosäuren sind wichtige chirale Ausgangsmaterialien für die organische Synthese z. B. von Pharmaka bzw. besitzen Bedeutung in der Human- und Tierernährung.

Die vorteilhafte Möglichkeit der stereoselektiven Spaltung der aus den entsprechenden Hydantoinen durch Hydantoinasen zugänglichen D,L-Carbamoylaminosäuren durch L-N- bzw. D-N-Carbamoylasen führte bisher zur Etablierung von Verfahren zur Herstellung von L- bzw. D-Aminosäuren im industriellen Maßstab. Für verschiedenste Mikroorganismen-Stämme wurde der Nachweis des Vorhandenseins von L-spezifischen Carbamoylasen erbracht (Ogawa et al., J. Mol. Cal. B: Enzym. 1997, 2, 163-176; Syldatk et al. in Drauz, K. und Waldmann, H.: Enzyme Catalysis in Organic Synthesis, Weinheim: VCH-Verlag, 1995).

Auch das Vorhandensein von L-N-Carbamoylase-Aktivität in Zellen des Stammes Arthrobacter aurescens ist schon seit längerem bekannt. Es ist darüber hinaus gelungen, diese Carbamoylase in homogener Form zu isolieren und die N-terminale Sequenz zu bestimmen (Lit. siehe Artikel; Müller, Doktorarbeit TU Braunschweig 1990). Das für das Enzym codierende Gen war allerdings unbekannt und ist dementsprechend bisher weder kloniert noch überexprimiert worden. Das rekombinantes Enzym bzw. dessen Aminosäuresequenz oder die diese Aminosäuresequenz codierende DNA waren somit in der Literatur nicht bekannt.

Der Literatur sind bisher drei rekombinant hergestellte L-Carbamoylasen zu entnehmen (Batisse et al., Appl. Environ. Microbiol. 1997, 63, 763-766; Mukohara et al., Biosci. Biotechn. Biochem. 1993, 57, 1935-1937; Watabe et al., J. Bacteriology 1992, 174, 962-969). Die dort genannten L-Carbamoylasen sind allerdings relativ instabil und somit für den Einsatz in einem großtechnischen Prozeß nicht geeignet.

Die L-spezifische Carbamoylase aus Arthrobacter aurescens (A.a.) konnte für die Spaltung von N-Carbamoylaminosäuren zu den freien L-Aminosäuren bisher nur als überwiegend homogen aufgereinigtes Enzym oder in Form von freien oder immobilisierten ruhenden ganzen Zellen eingesetzt werden. Beim letzteren Verfahren treten einige grundsätzliche Probleme auf. Dazu gehören u. a. Transportlimitierungen (besonders für die N-Carbamoyl-Aminosäuren), eventuelle Nebenreaktionen (Abbau der gebildeten Aminosäuren durch weitere vorhandene Enzyme) sowie Verunreinigungen, die durch Lysis der verwendeten ganzen Zellen das Produkt verunreinigen und aufwendige Reinigungsprozeduren notwendig machen.

In beiden zum Einsatz kommenden Formen ist die L-Carbamoylase aus A. a. sehr instabil, was im Falle der Benutzung von ruhenden immobilisierten auf das natürliche Protein-Turnover des Enzyms zurückgeführt wird (Siemann, Doktorarbeit TU Braunschweig 1992). Im Falle des Einsatzes als homogen gereinigtes Enzym wird die Labilität offensichtlich durch unvermeidbare Änderungen in der Proteinkonzentration und dem Salzgehalt bedingt. Des weiteren ist die Empfindlichkeit des Wild-Enzyms gegenüber Oxidationen beobachtet worden. Darüber hinaus erreicht man bei der Aufreinigung der Carbamoylase aus dem Wildstamm nur eine sehr geringe Ausbeute von 2,7% (Müller Doktorarbeit TU Braunschweig 1990). Diese geringe Ausbeute und das notwendige komplizierte Aufreinigungsverfahren sowie die bisher immanente Labilität des Enzyms sind prohibitiv für den Einsatz in einem konkurrenzfähigen industriellen Verfahren zur Gewinnung von L-Aminosäuren.

Aufgabe der vorliegenden Erfindung war es deshalb, eine verglichen mit den des Standes der Technik und den bisher aus Arthrobacter aurescens herstellbaren L-Carbamoylasen stabilere L-Carbamoylase in einfacherer Form und mit besserer Ausbeute zu erhalten. Dabei sollten allerdings die weiteren Anforderungen, die ein Verfahren im industriellen Maßstab an das eingesetzte Enzym stellt, wie z. B. Aktivität oder Selektivität bezüglich Substrat und Stereochemie, etc., nicht nachteilig beeinflußt werden.

Diese und weitere nicht näher genannte Aufgabe, welche sich jedoch ohne weiters aus dem Stand der Technik ergeben, werden durch das Enzyme, welche Gegenstand des Anspruchs 1 ist, gelöst. In Anspruch 2 sind die sich davon ableitenden Gensequenzen geschützt. Anspruch 3 richtet sich auf die mit den Gensequenzen modifizierten Vektoren, Anspruch 4 auf die entsprechenden Plasmide und Anspruch 5 auf die modifizierten Gastzellen.

Die Ansprüche 6 bis 9 beinhalten ein vorteilhaftes Verfahren zur Herstellung von L-Aminosäuren unter Verwendung des erfindungsgemäßen Enzymes.

Dadurch, daß man L-N-Carbamoylase aus Arthrobacter aurescens bzw. deren Mutanten rekombinant herstellt, gelangt man in sehr guten Ausbeuten von >90 % und ausgesprochen hoher Reinheit zu L-carbamoylasen, welche gegenüber den Carbamoylasen des Standes der Technik eine weitaus höhere Stabilität bei trotzdem guter Aktivität und sehr guter Stereoselektivität bzgl. der betrachteten Biotransformation aufweisen. Diese Tatsachen sind essentiell für den erfolgreichen Einsatz der Carbamoylasen in einem großtechnischen Verfahren zur Herstellung von L-Aminosäuren.

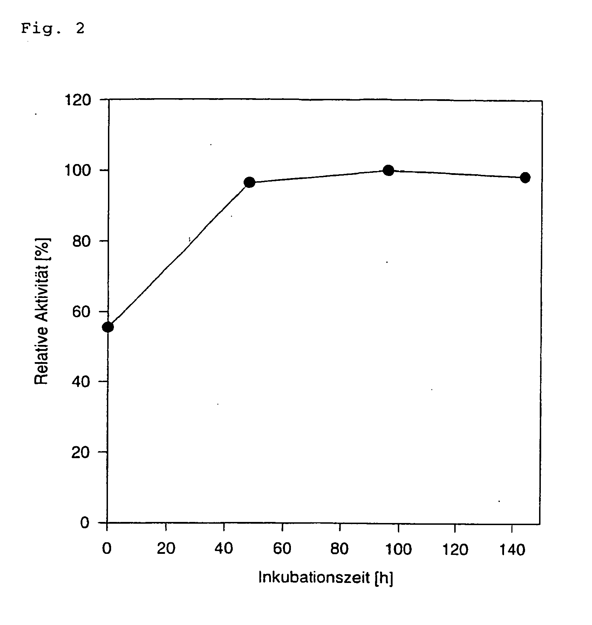

In Stabilitätsstudien hat sich gezeigt, daß die homogen gereinigte Carbamoylase bei 50 °C nur wenige Minuten Aktivität aufweist (Müller Doktorarbeit TU Braunschweig 1990), wohingegen die rec-Carbamoylase über Stunden aktiv ist. Bei 37 °C ist die Aktivität der rec-Carbamoylase über 100 h lang fast unverändert (Fig. 2). Dies war keineswegs vorherzusehen und ist dennoch um so vorteilhafter:

Darüber hinaus eröffnet der Einsatz dieser rekombinanten Carbamoylasen zum erstenmal die Möglichkeit, einen großtechnischen Zugang zu β-arylsubstituierten L-Aminosäuren durch eine enzymatische Biotransformation über (D,L)-N-Carbamoylaminosäuren zu erhalten. Die betrachtete Carbamoylase bietet als einzige der bisher bekannten Carbamoylasen neben der hohen L-Enantioselektivität auch die Möglichkeit der Umsetzung von β-arylsubstituierte L-N-Carbamoylaminosäuren in für ein großtechnisches Verfahren ausreichendem Maße. Zur Umsetzung mit den erfindungsgemäßen Enzymen eignen sich auch (D,L)-Formylaminosäuren dieser Provenienz. Aus diesen erhält man ebenfalls bevorzugt die freie L-Aminosäure.

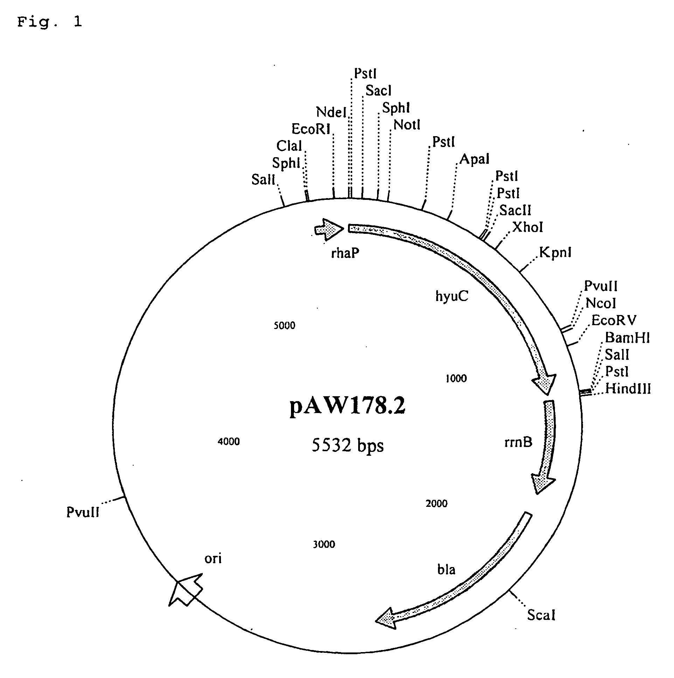

Die die rekombinante L-N-Carbamoylase aus Arthrobacter aurescens und deren Mutanten charakterisierenden Aminosäuresequenzen besitzen mit aus dem Stand der Technik bekannten Aminosäuresequenzen von L-N-Carbamoylasen lediglich eine Übereinstimmung von maximal 38 %. Die neuen und erfinderischen diese Aminosäuresequenzen codierenden Gensequenzen lassen sich nach an sich bekannten biochemischen Methoden herstellen. Ebenfalls neu und erfinderisch sind die diese Gensequenzen enthaltenden Vektoren, Plasmide bzw. Zellen von Gastorganismen. Bevorzugte Plasmide sind pAW 16 und pAW 178-2 (Fig. 1), ein bevorzugter Vektor ist pJOE 2702. Als Gastzellen können in Prinzip alle dem Fachmann bekannten und für diesen Zweck in Frage kommenden Mikroorganismen verwendet werden, bevorzugt ist jedoch E. coli JM 109 oder E. coli W3110.

Die Induktion der Expression kann prinzipiell mit allen dem Fachmann bekannten Methoden erreicht werden. Bevorzugt sind jedoch Rhamnose-, IPTG- sowie Lactose-Systeme.

Die Isolierung und Expression des L-N-Carbamoylase-Gens hyuC aus Arthrobacter aurescens erfolgt wie folgt.

Eine für das gesamte Genom von Arthrobacter aurescens repräsentativ Genbank in einem E. coli λRESIII-Vektor wurde erstellt und mit einem Oligonukleotid gescreent, das von der N-terminalen Aminosäuresequenz der gereinigten Hydantoinase aus Arthrobacter aurescens abgeleitet war. Dabei wurde das Plasmid pAW16 erhalten, dass ein 7.6 kb DNA-Fragment aus Arthrobacter aurescens enthielt. Dessen Nukleotidsequenz wurde vollständig bestimmt. Aus der Nucleotidsequenz wurde das Hydantoinase-Gen identifiziert. Am C-terminalen Ende des Hydantoinase-Gens hyuH wurde ein weiterer Leserahmen identifiziert, dessen abgeleitete Aminosäuresequenz Homologie zu einer N-L-Carbamoylase aus Pseudomonas sp. und Bacillus stearothermophilus zeigte. Weiterhin zeigte der Anfang des abgeleiteten Proteins Übereinstimmung mit dem durch Aminosäureseizuenzierung bestimmten N-terminalen Endes der aus Arthrobacter aurescens gereinigten L-N-Carbamoylase. Das Carbamoylase-Gen wurde über PCR aus pAW16 amplifiziert und in einen E. coli Expressionsvektor inseriert. Über einen durch den Zucker Rhamnose induzierbaren Promoter kann Carbamoylase in den rekombinanten Zellen in hoher Menge produziert werden.

Die Sequenzprotokolle Seq ID No 1 der DNA und Seq ID No 1 des Peptids geben die Nucleotidsequenz und die Aminosäuresequenz der rec-Carbamoylase aus A.a. wieder.

Unter Mutanten der L-Carbamoylase versteht der Fachmann gegebenenfalls durch Austausch einzelner Aminosäuren hergestellte sich jedoch von der rec-Carbamoylase aus A.a. ableitende Enzyme, welche sich bzgl. der oben angesprochenen Charakteristika in gleich guter oder sogar besserer Weise zum Einsatz in dem beschriebenen Verfahren eignen (bessere Aufarbeitungsmöglichkeit, geringere Oxidationsempfindlichkeit etc.). Unter Mutanten werden auch solche Enzyme verstanden, die durch Hinzufügen von Aminosäuren oder Aminosäuresequenzen zum rec-Enzym entstehen. Bevorzugt sind dabei solche, die eine His-TAG oder Asp-TAG Modifikation am C-Terminus aufweisen.

Erfindungsgemäß werden mit diesen vorteilhaften L-N-Carbamoylasen aus den entsprechenden L-N-Carbamoyl-Aminosäuren bzw. L-N-Formyl-Aminosäuren die gewünschten L-Aminosäuren hergestellt. Wie schon gesagt, weisen die beanspruchten L-N-Carbamoylasen eine bevorzugte Umsetzung von β-arylsubstituierten L-N-Carbamoyl- bzw. L-N-Formylaminosäuren auf. Diese chiralen Verbindungen können aus chiralen Hydantoinen bevorzugt per Hydantoinasen hergestellt werden. Durch den zusätzlichen-Einsatz eines chemischen oder enzymatischen Verfahrens zur Racemisierung von Hydantoinen gelingt es äußerst vorteilhaft, aus leicht herstellbaren Hydantoinen einen großtechnisch wertvollen Prozeß zur Gewinnung von L-Aminosäuren zu konstruieren. Bevorzugt kommen dabei chirale 5-monosubstituierte Hydantoine mit aromatischen Seitenketten in Frage. Die Stereoisomere dieser Hydantoine werden durch den vorgelagerten Racemisierungsschritt - ggf. durch eine Racemase - stetig equilibriert, während die Hydantoinase die entsprechenden L- oder D,L-N-Carbamoylaminosäuren hervorbringt. Die stereospezifischen erfindungsgemäßen rec-L-N-Carbamoylasen besorgen dann den letzten Schritt zur Herstellung der L-Aminosäure, da die Hydantoinase aus A.a. im Gegensatz zur Carbamoylase keine absolute Stereospezifität zeigt. Durch die vorangestellte stetige Equilibrierung der Stereoisomere der Hydantoine wird gewährleistet, daß sämtliches Hydantoin, ob D- oder L-konfiguriert, schlußendlich zu einer L-Aminosäure umgesetzt wird.

Bevorzugt setzt man die geschützten Aminosäuren mit den Enzymen im sogenannten Enzym-Membran-Reaktor um. Durch die Stabilität der eingesetzten Enzyme ist gewährleistet, daß die durch eine Ultrafiltrationsmembran im Reaktor zurückgehaltenen Enzyme mehrmals (>10 mal) hintereinander zur L-Aminosäureproduktion eingesetzt werden können. Damit werden Kosten und Aufwand für das Verfahren gespart, da lediglich Substrat zudosiert und Produkt abgezogen werden muß.

Alternativ kann das Enzym auf Trägern immobilisiert werden. Dies gelang in genügend hoher Ausbeute erst, nachdem das rec-Enzym in reiner Form eingesetzt werden konnte. Es können all dem Fachmann bekannten (kovalenten, adsorptiven, etc.) Methoden zur Immobilisierung von Enzymen auf Trägermaterialien (Kieselgele, SiO₂, EAH-Sepharose, Nitrocellulose, Eupergit^{®}) verwendet werden, vorteilhaft ist jedoch die carbodiimidvermittelte kovalente Kopplung an EAH-Sepharose. So modifiziert kann das Enzym auch im Festbettreaktor verwendet werden.

Die folgenden Beispiele sollen die Erfindung erläutern, sie jedoch keinesfalls einschränken.

### Beispiele:

### Isolierung der Gesamt-DNA aus Arthrobacter aurescens:

180 ml des Hefeextraktmediums K2 (Bacto-Pepton 4,0 g, Hefeextrakt, 4,0 g, Glycin, 7,5 g, KH₂PO₄ 2.0 g, K₂HPO₄ · H₂O 4.0 g, pH 7,0 in 1000 ml H₂O) wurde mit Arthrobacter aurescens angeimpft und bei 30°C unter Schütteln (200 rpm) inkubiert. Die Zellen wurden geerntet durch Zentrifugation in einem Sorvall GSA-Rotor bei 4 °C, 6000 rpm, 10 min, das Pellet in 20 ml TE-Puffer (10 mM Tris-HCl, 1 mM EDTA, pH 8,0) gewaschen, erneut zentrifugiert wie vorher und in 16 ml TES-Puffer (25 mM Tris-HCl, 25 mM EDTA, 10% Sucrose, pH 8,0) resuspendiert. Zur Zellyse wurden 2 ml Lysozymlösung (100 mg/ml TES-Puffer) zugegeben und 60 min bei 37°C inkubiert. Anschließend wurden 2 ml 10 % Na-Lauryl-Sarcosinat und 2 ml Proteinase K (10 mg/ml) zugegeben und weitere 60 min bei 37 °C inkubiert. Dann wurden 20 g CsCl und 0.6 ml Ethidiumbromid (10 mg/ml) zugegeben und bei 35000 rpm, 17 °C , T1270-Rotor 48 Stunden in einer Sorvall-Ultrazentrifuge zentrifugiert. Die DNA wurde über UV-Licht identifiziert, über eine Einwegspritze aus dem Zentrifugenröhrchen abgezogen und für 2 h gegen 2 Liter TE-Puffer dialysiert. Das Ethidiumbromid wurde durch zweimalige Phenolextraktion mit jeweils 2 ml neutralisiertem Phenol entfernt und die DNA-Lösung erneut zweimal gegen jeweils 2 L TE-Puffer dialysiert.

### Konstruktion der λ Genbank:

10 µg genomischer DNA von Arthrobacter aurescens wurden mit 0,75 Units des Restriktionsenzyms XhoII für 30 min bei 37 °C behandelt. Dabei wird die genomische DNA nur partiell geschnitten. Die DNA wurde in einem 0.7%igen "Low melting agarose gel" aufgetrennt, die DNA durch UV-Licht sichtbar gemacht und ein Agaroseblock mit Fragmenten der Größe zwischen 7 kb auf 13 kb ausgeschnitten. Der Agaroseblock wurde bei 65°C geschmolzen in einem Volumen von 500 µl Puffer (TE-Puffer mit 0.5 M NaCl), mit gleicher Menge Phenol extrahiert und Agarose und Phenol durch Zentrifugation bei 4 °C in einer Eppendorfzentrifuge bei 13000 rpm, 30 min, 4 °C abgetrennt. Die wässrige Phase wurde mit einem Zehntel Volumen 3 M Na-Acetat, pH 6,2 und 2,5 Volumen Ethanol versetzt, 30 min bei -70 °C inkubiert, 10 min zentrifugiert in einer Eppendorfzentrifuge, die Flüssigkeit entfernt und die gefällte DNA mit 100 µl 70 % Ethanol gewaschen, an Luft getrocknet und in 10 µl TE-Puffer resuspendiert. Die λRESIII-Vektor DNA wurde wie in Sambrook et al. beschrieben präpariert. Der Phage ist ein Substitutionsvektor, d. h. ein Restriktionsfragment wird aus der Vektor-DNA entfernt und durch ein anderes Fragment ersetzt. Ohne Insert ist der Phage nicht vermehrungsfähig (Altenbuchner, 1993). In diesem Fall wurde ein 9 kb BamHI-Fragment mit den bakteriellen Lux-Genen aus dem Vektor entfernt, indem 1 µg Vektor-DNA mit 10 Unit BamHI 1 h behandelt wurde, die DNA über "Low melting agarose" aufgetrennt und die DNA der λ-Arme wie oben beschrieben über das Gel gereinigt wurde. Die DNAs wurden anschließend ligiert, indem jeweils circa 25 ng der beiden Vektor-Arme mit 25 ng XhoII geschnittener Fragmente von A. aurescens in einem Volumen von 10 µl, Ligasepuffer des Herstellers (Boehringer GmbH) und 0,5 Unit Ligase über Nacht bei 17 °C inkubiert wurden. 2 µl des Ansatzes wurden in ein in vitro-Verpackungssystem eingesetzt und in Phagenpartikel verpackt. Der Verpackungsansatz war wie in Sambrook et al. beschrieben aus den beiden *E. coli* Stämmen *E. coli* BHB2690 (Sonifizierter Extract, SE) und BHB2688 (Frier-Auftau-Extrakt FE) hergestellt. Zur Verpackung wurden 7 µl Puffer A (20 mM Tris-HCl, 3 mM MgCl₂, 0.05 %(V/V) 2-Mercaptoethanol, 1 mM EDTA, pH 8.0), 10 µl FE-Extrakt, 6 µl SE-Extrakt, 1 µl Puffer M1 (113 µl H₂O, 3 µl 1 M Tris-HCl, pH 7,5, 300 µl 0,05 M Spermin, 0.1 M Putrescin, 75 µl 0,1 M ATP, 1 µl 2 Mercaptoethanol, 9 µl 1 M MgCl₂) und 2 µl DNA aus dem Ligationsansatz gemischt und 1 h bei 25°C inkubiert. Durch Zugabe von 0,5 ml SM-Puffer (5,8 g NaCl, 2 g MgSO₄ ·7 H₂O, 50 ml 1 M Tris-HCl, pH 7,5, 2 g Gelatine, pro 1 L) und einem Tropfen Toluol wurde die Verpackung gestoppt.

### Identifizierung eines rekombinanten Phagen mit Hydantoinase-Gen durch Plaque-Hybridisierung:

Das Phagenlysat, das aus der in vitro Verpackung erhalten wurde, wurde in SM-Puffer auf circa 5000 Phagen/ml verdünnt. 0.1 ml verdünntes Phagenlysat wurde mit 0.1 ml Übernachtkultur in LBₘₐₗ des E. coli-Stammes TAP90 für 5 min bei Raumtemperatur inkubiert, in 3 ml LBₘₐₗ -Weichager aufgenommen und auf eine LB-Platte gegossen und bei 37°C über Nacht inkubiert. (LBₘₐₗ: 10 g Bacto-Trypton, 5 g Hefeextrakt, 10 g NaCl, 2 g Maltose, pro 1 Liter H₂O, pH 7,5; LB-Agarplatten: wie LBₘₐₗ ohne Maltose mit 1,5 % Agar, LBₘₐₗ -Weichagar wie LBₘₐₗ mit 0.7 % Agar). In dieser Zeit entwickelten sich sichtbare Plaques auf der Platte. Auf die Agarplatte wurde ein Nylonfilter (Quiabrane, Quiagen GmbH) aufgelegt und dadurch die Phagen-DNA in den Plaques auf den Filter übertragen. Die DNA auf dem Filter wurde durch Auflegen des Filters auf einen Stapel Whatmann-Papier, der mit Denaturierungslösung (0,5 M NaOH, 1,5M NaCl) getränkt war, 5 min denaturiert und auf gleiche weise 5 min renaturiert mit der Renaturierungslösung (1,5 M NaCl, 0,5 M TrisHCl, pH 7,4). Der Filter wurde in 2xSSC (1xSSC: 8,75 g NaCl, 4,4 g NaCitrat pro 1 L H₂O, pH 7,0) gewaschen, getrocknet und 30 min bei 120 °C in einem Vakuumofen gebacken, um die DNA zu fixieren. Der Filter wurde dann vorhybridisiert mit Hybridisierungslösung (5xSSC, 1% Blocking Reagenz von Boehringer Mannheim, 0,1 % Na-Lauryl-Sarcosinat, 0.02 % Natriumdodecylsulfonat) bei 32 °C. Nach zwei Stunden wurde radioaktiv markiertes Oligonucleotid zugegeben. Das Oligonukleotid wurde von der Firma MWG Biotech GmbH bezogen, die Nukleotidsequenz wurde aus der N-terminalen Aminosäuresequenz der L-Hydantoinase aus Arthrobacter aurescens (Siemann, Dissertation 1992) abgeleitet.

Oligonukleotid: 5'-ATGTT(C/T)GA(T/C)GT(A/C/T/G)AT(A/C/T)GT-3'
10 pMol dieses Oligonukleotids wurden mit 80µCi γ-³²P-ATP und 6 Unit Polynukleotidkinase in einen volumen von 10 µl
30 min bei 37°C markiert und das markierte oligonukleotid zur Hybridisierlösung gegeben. Hybridisiert wurde bei 32 °C
20 Stunden. Der Filter wurde zunächst kurz mit 0,2xSSC, 0.1 % SDS gewaschen bei Raumtemperatur, 3C min bei 32 °C
und ein weiteres Mal mit 0,1xSSC, 0,1 % SDS 15 min bei 32°C. Nach dem Trocknen wurden über Autoradiographie positive Phagen ermittelt. Dabei wurden aus 2500 Phagen zwei isoliert, die mit der Sonde hybridisierten. Einer der Phagen wurde weiter untersucht.

### Umwandlung des rekombinanten Phagen in ein Plasmid:

Der Vektor λRESIII enthält neben den λ-Genen und dem Substitutionsfragment einen Replikationsursprung des E. coli-Plasmids Rtsl, ein Kanamycinresistenz-Gen sowie zwei Erkennungssequenzen (res) für ein ortsspezifisches Rekombinationssystem (der Resolvase aus dem Transposon Tn1721). Durch Infektion eines spezifischen E. coli-Stamms (E. coli HB101 F'Lac [: : Tn1739 tnpR]), der ein Derivat dieses Transposons enthält, wird der Phage in seiner Vermehrung gehemmt und gleichzeitig wird das Plasmid zusammen mit dem Substitutionsfragment über die Transposonkodierte Resolvase aus der Phagen-DNA herausgeschnitten und in ein Plasmid umgewandelt. Rekombinante Phagen wurden aus dem Plaque isoliert, der mit der Sonde hybridisierte, indem der Plaquebereich aus der Agarplatte ausgestochen wurde. Das Material wurde in 0.5 ml SM-Puffer resuspendiert und 0,1 ml dieser Phagensuspension wurde mit 0.1 ml einer Übernachtkultur des Stammes E. coli HB101 F'Lac[::Tn1739 tnpR] gemischt, 5 min bei Raumtemperatur inkubiert, in 2 ml LB-Medium mit 0.1 M Isopropyl-β-Thiogalactopyranosid gegeben und für 45 min auf einem Roller bei 37°C inkubiert. Anschließend wurden Aliquots auf LB-Agarplatten mit 50 µg/ml Kanamycin plattiert und die Agarplatten bei 37°C über Nacht inkubiert. Von den erhaltenen Kolonien wurde Plasmid-DNA isoliert nach einer von Kieser (1984) beschriebenen Methode und dabei das Plasmid pAW16 erhalten. pAW16 enthält ein 7,6 kb großes Fragment aus Arthrobacter aurescens. Das Fragment wurde mit Hilfe eines ALF-Sequencers und dem AutoRead^{™} Sequencing Kits der Firma Pharmacia vollständig sequenziert und mit den Programmen des GCG-Software-Pakets analysiert. Ein offener Leserahmen konnte aufgrund der Identität der abgeleitetes Aminosäuresequenz mit dem sequenzierten N-terminalen Ende der Hydantoinase als Gen hyuH für Hydantoinase identifiziert werden. Ein weiterer offener Leserahmen wurde am Ende von hyuH identifiziert, beginnend mit einem GTG-Startcodon eine Base vor dem Stopcodon TGA des hyuH Gens. Die abgeleitete Aminosäuresequenz dieses offenen Leserahmens zeigt Homologie zu einer N-L-Carbamoylase von Bacillus stearothermophilus NS1122A (Mukohara et al., 1993) und von Pseudomonas sp. NS671 (Watabe et al., 1992). weiterhin stimmt die N-terminale Aminosäuresequenz mit der N-terminalen Sequenz der N-L-Carbamoylase aus A.s überein (Siemann, Dissertation 1992). Das Gen wurde mit hyuC für N-L-Carbamoylase bezeichnet.

### Expression von hyuC in E. coli JM109 über einen mit Rhamose induzierbaren Promotor:

zur Expression des N-L-Carbamoylase-Gens hyuC in E. coli wurde das Gen über die Polymeraseketten-Reaktion (PCR) amplifiziert. Die beiden Primer S956 und S957 wurden von MWG Biotech GmbH bezogen.
S956: 5'-AGAACATATGTTTGACGTAATAGTTAAGAA-3'
S957: 5'-AAAAGGATCCTCACTTCGACGCCTCGTA-3'

Durch die Primer wurde an den N-Terminus eine Restriktionsschnittstelle für das Enzym NdeI eingefügt und am C-Terminus eine BamHI-Schriittstelle. Beide Schnittstellen sind nötig, um das Gen hinter den Rhamnosepromotor des Vektors pJOE2702 (Volff et al., 1996) zu inserierten. Weiterhin wurde das in Arthrobacter aurescens für die Translation des Gene benutzte GTG Startcodon in ein ATG Startcodon umgewandelt.

Der Ansatz wurde in 100 µl durchgeführt. Eingesetzt wurden 1 ng pAW16 DNA, je 30 pMol der beiden Primer, 0,2 mM dNTP, 16 mM (NH₄)₂SO₄, 67 mM Tris-HCl, pH 8,8, 0.01 % Tween, 1,5 mM MgCl₂, 0,5 Unit Taq-Polymerase. Die Amplifikation erfolgte in 30 Zyklen mit drei Stufen, 1 min 94°C, 1 min 50°C und 1,5 min 72°C in einem MiniCycler PTC150, Biozym Diagnostik GmbH. Das amplifizierte Fragment wurde über eine QUIAquick Spin Säule (Quiagen GmbH) gereinigt. 500 ng des Fragments wurde in einem Volumen von 20 µl mit jeweils 10 Unit der Restriktionsenzyme NdeI und BamHI in den vom Hersteller mitgeliefertem Puffer für 1 h behandelt. Ebenso wurde unter gleichen Bedingungen DNA des Vektors pJOE2702 mit diesen Enzymen behandelt. Die DNAs wurden über ein 0,7 % Agarose-Gel aufgetrennt und wie oben beschrieben die Fragmente aus dem Gel isoliert. Jeweils 20 ng PCR-Fragment und 20 ng Vektor wurden unter den oben beschriebenen Bedingungen ligiert. Mit der ligierten DNA wurde E. coli JM109 nach einem Protokoll von Chung et al., (1989) transformiert. Die Zellen wurden auf LB-Agarplatten plattiert, die 100 µg/ml Ampicillin enthielten (LBₐₘₚ). Am nächsten Tag wurden aus Ampicillin-resistenten Kolonien Plasmide isoliert und mit den Restriktionsenzymen NdeI und BamHI charakterisiert. Das Plasmid pAW178-2 enthielt das hyuC-Gen in der gewünschten Orientierung unter dem Rhamnose-Promoter.

### Expression von hyuC in E. coli JM109/pAW178-2:

1 ml einer Übernachtkultur von E. coli JM109/pAW178-2 (Fig. 1) in LBₐₘₚ wird in 100 ml frisches LBₐₘₚ-Medium in einem 1 Liter Erlenmeyerkolben gegeben und dieser bei 30 °C in einem Wasserbad unter Schütteln (200 rpm) inkubiert. Bei einer optischen Dichte der Kultur von 0.5 (gemessen bei einer Wellenlänge von 600 nm in einem Photometer) werden 1 ml einer 20%igen Rhamnoselösung zur Kultur gegeben (Endkonzentration 0,2 g/L) und die Kultur weiter inkubiert, Nach weiteren 9 h Inkubation wird etwa das Maximum der N-L-Carbamoylase-Aktivität erreicht, eine spezifische Aktivität von 1,8 Units/mg Rohextrakt, gemessen mit N-Carbamoyl-L-Tryptophan als Substrat. Keine Aktivität zeigte sich in Kulturen, die nicht mit Rhamnose induziert worden waren.

### Bestimmung des Substratspektrums der rec-L-N-Carbamoylase aus A.a.

Die Substratspezifität der Carbamoylase wurde mit homogen gereinigtem Enzym untersucht. Dieses wurde nach Zellaufschluß und Ionenaustauch-Chromatographie an Streamline-DEAE- und MonoQ-Medien erhalten. Es wurde eine Lösung des jeweiligen Substrates in einer Konzentrationen von 1.8 mM (Ausnahme N-Carbamoyl-L-Methionin: 50 mM) in 0.1 M Tris-HCl, pH 8.5 hergestellt und anstelle des Standardsubstrates im Standardaktivitätstest verwendet. Anstelle der im Standardtest zum Stoppen verwendeten Trichloressigsäure (TCA) wurde im Falle der Substrate N-Formyl-D,L-Tryptophan, N-Carbamoyl-L-Thienylalanin und N-Acetyl-D,L-Tryptophan mittels Hitzedenaturierung (Inkubation für 10 min im kochenden Wasserbad) und im Falle des Substrates N-Carbamoyl-D,L-Phenylalanin 900 µl Methanol zu 100 µl Assaylösung verwendet. Anschließend wurde die Konzentration der Aminosäure entweder mittels HPLC bzw. colorimetrisch nach Reaktion mit Ninhydrin oder per HPLC bestimmt.

**Tab. 1 Bestimmung des Substratspektrums und der Stereospezifität der rec-L-N-Carbamoylase aus A.a.**

| Substrat | Produkt | Aktivität | Kₘ | Analytische Methode |
|---|---|---|---|---|
| | | % | [mM] | |
| N-carbamoyl-L-Tryptophan | L-Tryptophan | 100 | 0.12 | HPLC colorimetrisch |
| N-Carbamoyl-D-Tryptophan | D-Tryptophan | n.n. | | HPLC |
| N-Formyl-D-L-Tryptophan | L-Tryptophan | 97 | 8.5 | HPLC |
| N-Acetyl-D,L-Tryptophan | L-Tryptophan | n.n. | | HPLC |
| N-Carbamoyl-L-Thienylalanin | L-Thienylalanin | 314 | 2.3 | HPLC colorimetrisch |
| N-Carbamoyl-D,L-Phenylalanin | L-Phenylalanin | 97 | 0.7 | HPLC |
| N-Carbamoyl-D-Phenylalanin | D-Phenylalanin | n.n. | | HPLC |
| N-Carbamoyl-L-Tyrosin | L-Tyrosin | 126 | 0.3 | HPLC |
| N-Carbamoyl-L-Methionin | L-Methionin | 17 | | HPLC colorimetrisch |
| N-Carbamoyl-L-Phenylglycin | L-Phenylglycin | n.n. | | HPLC colorimecrisch |
| N-Carbamoyl-D-Phenylglycin | D-Phenylglycin | n.n. | | colorimetrisch |
| N-Carbamoyl-β-Alanin | β-Alanin | n.n. | | colorimetrisch |
| N-Carbamoyl-D,L-Aspartat | L-Aspartat | n.n. | | colorimetrisch |
| N-Formyl-L-Aspartat | L-Aspartat | n.n. | | colorimetrisch |
| N- Carbamoyl-L-Glutamat | L-Glutamat | n.n. | | colorimetrisch |
| N- Carbamoyl-L-Arginin | L-Arginin | n.n. | | colorimetrisch |

| | | | | |
|---|---|---|---|---|
| n.n: nicht nachweisbar | | | | |

### Temperaturstabilität der rec-L-N-Carbamoylase aus A.a.

Die homogen gereinigte rekombinante L-Carbamoylase wurde bei 37 °C gelagert. In Zeitabständen von 1 - 100 h wurde jeweils der Standardtest zur Aktivitätsbestimmung durchgeführt. Die anfangs gemessene Aktivität blieb über 100 h nahezu unverändert (Fig. 2).

### Immobilisierung der rec-L-N-Carbamoylase:

250 µl Enzymlösung (nach der Reinigung, Proteinkonzentration: 2.3 mg/ml) werden mit 19.75 ml einer Lösung von 1 mM MnCl₂ in Wasser verdünnt (Proteinendkonzentration: 0.028 mg/ml). Von dieser Lösung werden 4 ml zu 1.5 g der zuvor gewaschenen EAH-Sepharose 4B (von Pharmacia Biotech, Waschen nacheinander mit 0.1 M Phosphatpuffer, pH 6.5 und anschließend mit einer Lösung von 1 mM MnCl₂ in Wasser) gegeben. Nach einer Adsorptionsphase von 20 min werden 545 µl einer Kopplungslösung von 500 mM N-(3-Dimethylaminopropyl)-N'-ethyl-carbodiimid-hydrochlorid (EDC) in Wasser, pH 6.0 zu dem Ansatz gegeben (Endkonzentration EDC: 60 mM) und im Überkopfschüttler bis zu 30 h (vorzugsweise 15 h) geschüttelt. Es wird abgenutscht und dreimal mit 0.2 M Tris-Puffer, 0.5 M NaCl, pH 7.0 gewaschen. In der letzten Waschlösung wird das Immobilisat eine Stunde lang aufbewahrt. Anschließend wird erneut gewaschen (0.1 M Tris-Puffer, pH 7.0) und in 0.1 M Tris-Puffer, 0.1 mM MnCl₂, pH 7.0 gelagert). Die Aktivität des immobilisierten Enzyms betrug 100 % verglichen mit der des eingesetzten.

### Enzymmessungen der N-L-Carbamoylase:

5 ml einer mit Rhamnose induzierten Zellkultur werden bei 3000 rpm für 5 min in einer Heraeus Megafuge zentrifugiert, mit 5 ml Puffer (200 mM Tris-HCl, pH 7,0) gewaschen, erneut zentrifugiert, in 1 ml Puffer (200 mM Tris-HCl, 0,1 mM MnCl₂ pH 7,0) aufgenommen und durch Ultraschall aufgeschlossen (Ultrasonics Sonicator, Mikrotip, 2x30 sec, duty cycle 50 %). Zelltrümmer werden durch Zentrifugation in einer Eppendorfzentrifuge bei 13000 rpm, 10 min entfernt. Der Überstand, als Rohextrakt bezeichnet, wird für die Enzymmessung eingesetzt. 100 µl Rohextrakt oder aufgereinigtes Enzym werden mit auf 37°C vorgewärmten 800 µl 2 mM N-Carbamoylaminosäure in 100 mM Tris-HCl, pH 8,5 gemischt und 10 min bei 37°C inkubiert. Die Reaktion wird durch Zugabe von 400 µl Trichloressigsäure gestoppt. Das ausgefallene Protein wird durch Zentrifugation in einer Eppendorfzentrifuge bei 13000 rpm für 10 min abgetrennt. Der Überstand wird anschließend in einer HPLC oder colorimetrisch (Ninhydrin) vermessen. Die Auftrennung des Reaktionsgemisches erfolgt in einer HPLC mit einer RP-18 Säule, Laufmittel ist 0,3 % (V/V) Phosphorsäure, 80 % Methanol, Flußrate 1,0 ml/min. Die Detektion erfolgt durch Messung der UV-Absortion bei 280 nm Wellenlänge.

## Patentansprüche

1. rec-L-N-Carbamoylase aus Arthrobacter aurescens gemäß Seq. ID No. 1.

2. Gensequenzen, die die Aminosäuresequen nach Anspruch 1 codieren.

3. Vektoren, aufweisend die Gensequenzen nach Anspruch 2.

4. Plasmide, aufweisend die Gensequenzen nach Anspruch 2.

5. Gastzellen, aufweisend die Gensequenzen nach Anspruch 2.

6. Verfahren zur Herstellung von L-Aminosiauren mittels rec-L-N-Carbamoylase nach Anspruch 1

7. Verfahren nach Anspruch 6,
**dadurch gekennzeichnet,**
**daß** man N-Carbamoyl- bzw. N-Formylaminosäuren umsetzt.

8. Verfahren nach Anspruch 7,
**dadurch gekennzeichnet,**
**daß** man die N-Carbamoylaminosauren mittels Hydantoinasen aus den entsprechenden Hydantoinen herstellt.

9. Verfahren nach Anspruch 8,
**dadurch gekennzeichnet,**
**daß** man die eingesetzten Hydantoine durch enzymatische oder chemische Verfahren stetig racemisiert.

## Claims

1. rec-L-N-Carbamoylase derived from *Arthrobacter aurescens,* as set forth in Seq. ID No. 1.

2. Gene sequences encoding the amino acid sequence according to Claim 1.

3. Vectors having the gene sequences according to Claim 2.

4. Plasmids having the gene sequences according to Claim 2.

5. Guest cells having the gene sequences according to Claim 2.

6. Method for producing L-amino acids by means of rec-L-N-carbamoylase according to Claim 1.

7. Method according to Claim 6,
**characterized in that**
N-carbamoylamino acids or N-formylamino acids are converted.

8. Method according to Claim 7,
**characterized in that**
the N-carbamoylamino acids are produced from the corresponding hydantoins by means of hydantoinases.

9. Method according to Claim 8,
**characterized in that**
the hydantoins used are constantly racemized by enzymic or chemical methods.

## Revendications

1. rec-L-N-carbamoylase d'*Arthrobacter aurescens* selon Seq. ID No. 1.

2. Séquences de gènes qui codent pour les séquences d'aminoacides selon la revendication 1.

3. Vecteurs, comportant les séquences de gènes selon la revendication 2.

4. Plasmides, comportant les séquences de gènes selon la revendication 2.

5. Cellules hôtes, comportant les séquences de gènes selon la revendication 2.

6. Procédé pour la production de L-aminoacides au moyen de la rec-L-N-carbamoylase selon la revendication 1.

7. Procédé selon la revendication 6, **caractérisé en ce qu'**on met en réaction des N-carbamoyl- ou N-formylaminoacides.

8. Procédé selon la revendication 7, **caractérisé en ce qu'**on prépare les N-carbamoylaminoacides à l'aide d'hydantoïnases, à partir des hydantoïnes correspondantes.

9. Procédé selon la revendication 8, **caractérisé en ce que** les hydantoïnes utilisées sont toujours racémisées par des procédés chimiques ou enzymatiques.


### SEQUENZPROTOKOLL

(1) ALLGEMEINE ANGABEN:
   (i) ANMELDER:
      (A) NAME: Degussa Aktiengesellschaft
      (B) STRASSE: Weissfrauenstrasse 9
      (C) ORT: Frankfurt
      (D) BUNDESLAND: Hessen
      (E) LAND: Deutschland
      (F) POSTLEITZAHL: 60311
      (G) TELEFON: 06181593924
      (H) TELEFAX: 06181594304

      (A) NAME: Universitaet Stuttgart
      (B) STRASSE: Allmandring 31
      (C) ORT: Stuttgart
      (E) LAND: Deutschland
      (F) POSTLEITZAHL: 70569
      (G) TELEFON: 07116855156
      (H) TELEFAX: 07116855164

      (A) NAME: Boehringer Mannheim GmbH
      (B) STRASSE: Sandhofer Strasse 116
      (C) ORT: Mannheim
      (E) LAND: Deutschland
      (F) POSTLEITZAHL: 68298
      (G) TELEFON: 06217594267
      (H) TELEFAX: 06217594461
   (ii) BEZEICHNUNG DER ERFINDUNG: Rekombinante L-N-Carbamoylase aus Arthrobacter aurescens, Verfahren zur Herstellung von L-Aminosaeuren mittels dieser
   (iii) ANZAHL DER SEQUENZEN: 1
   (iv) COMPUTER-LESBARE FASSUNG:
      (A) DATENTRAEGER: Floppy disk
      (B) COMPUTER: IBM PC compatible
      (C) BETRIEBSSYSTEM: PC-DOS/MS-DOS
      (D) SOFTWARE: **PatentIn** Release #1.0, Version #1.30 (EPA)
(2) ANGABEN ZU SEQ ID NO: 1:
   (i) SEQUENZKENNZEICHEN:
      (A) LAENGE: 1239 Basenpaare
      (B) ART: Nucleotid
      (C) STRANGFORM: Einzelstrang
      (D) TOPOLOGIE: linear
   (ii) ART DES MOLEKÜLS: Genom-DNA
   (iii) HYPOTHETISCH: NEIN
   (iv) ANTISENSE: NEIN
   (v) ART DES FRAGMENTS: N-Terminus
   (vi) URSPRÜNGLICHE HERKUNFT:
      (A) ORGANISMUS: Arthrobacter
      (B) STAMM: aurescens
   (viii) POSITION IM GENOM:
      (C) EINHEITEN: bp
   (xi) SEQUENZBESCHREIBUNG: SEQ ID NO: 1:
   GTGACCCTGC AGAAAGCGCA AGCGGCGCGC ATTGAGAAAG AGATCCGGGA GCTCTCCCGG      60
   TTCTCGGCAG AAGGCCCCGG TGTTACCCGG CTGACCTACA CTCCAGAGCA TGCCGCCGCG     120
   CGGGAAACGC TCATTGCGGC TATGAAAGCG GCCGCCTTGA GCGTTCGTGA AGACGCACTC     180
   GGAAACATCA TCGGCCGACG TGAAGGCACT GATCCGGAGC TTCCTGCGAT CGCGGTCGGT     240
   TCACACTTCG ATTCTGTCCG AAACGGCGGG ATGTTTGATG GCACTGCAGG CGTGGTGTGC     300
   GCCCTTGAGG CTGCCCGGGT GATGCTGGAG AACGGCTACG TGAATCGGCA TCCATTTGAG     360
   TTCATCGCGA TCGTGGAGGA GGAAGGGGCC CGCTTCAGCA GTGGCATGTT GGGCGGCCGG     420
   GCCATTGCAG GGTTGGTCGC CGACAGGGAA CTGGACTCTT TGGTTGATGA GGATGGAGTG     480
   TCCGTTAGGC AGGCGGCTAC TGCCTTCGGC TTGAAGCCGG GCGAACTGCA GGCTGCAGCC     540
   CGCTCCGCGG CGGACCTGCG TGCTTTTATC GAACTACACA TTGAACAAGG ACCGATCCTC     600
   GAGCAGGAGC AAATAGAGAT CGGAGTTGTA ACCTCCATCG TTGGCGTTCG CGCATTGCGG     660
   GTTGCCGTCA AAGGCAGAAG CGACCACGCC GGCACAACCC CCATGCACCT GCGCCAGGAT     720
   GCGCTGGTAC CCGCCGCTCT CATGGTGAGG GAGGTCAACC GGTTCGTCAA CGAGATCGCC     780
   GATGGCACAG TGGCTACCGT TGGCCACCTC ACAGTGGCCC CCGGTGGAGG CAACCAGGTC     840
   CCGGGGGAGG TGGACTTCAC ACTGGACCTG CGTTCTCCGC ATGAGGAGTC GCTCCGCGTG     900
   CTGATCGACC GCATCTCGGT CATGGTCGGC GAGGTCGCCT CCCAGGCCGG TGTGGCTGCC     960
   GATGTGGATG AATTTTTCAA TCTCAGCCCG GTGCAGCTGG CTCCTACCAT GGTGGACGCC    1020
   GTTCGCGAAG CGGCCTCGGC CTTGCAGTTC ACACACCGGG ATATCAGCAG TGGGGCGGGC    1080
   CACGACTCGA TGTTCATCGC CCAGGTCACG GACGTCGGAA TGGTTTTCGT TCCAAGCCGT    1140
   GCTGGCCGGA GCCACGTTCC CGAAGAATGG ACCGATTTCG ATGACCTTCG CAAAGGAACT    1200
   GAGGTTGTCC TCCGGGTAAT GAAGGCACTT GACCGGTAA                           1239

### SEQUENZPROTOKOLL

(1) ALLGEMEINE ANGABEN:
   (i) ANMELDER:
      (A) NAME: Degussa Aktiengesellschaft
      (B) STRASSE: Weissfrauenstrasse 9
      (C) ORT: Frankfurt
      (D) BUNDESLAND: Hessen
      (E) LAND: Deutschland
      (F) POSTLEITZAHL: 60311
      (G) TELEFON: 06181593924
      (H) TELEFAX: 06181594304

      (A) NAME: Universitaet Stuttgart
      (B) STRASSE: Allmandring 31
      (C) ORT: Stuttgart
      (E) LAND: Deutschland
      (F) POSTLEITZAHL: 70569

      (A) NAME: Boehringer Mannheim GmbH
      (B) STRASSE: Sandhofer Strasse 31
      (C) ORT: Mannheim
      (E) LAND: Deutschland
      (F) POSTLEITZAHL: 68298
      (G) TELEFON: 06217594267
      (H) TELEFAX: 06217594461
   (ii) BEZEICHNUNG DER ERFINDUNG: Rekombinante L-N-Carbamoylase aus Arthrobacter aurescens, Verfahren zur Herstellung von L-Aminosaeuren mittels dieser
   (iii) ANZAHL DER SEQUENZEN: 1
   (iv) COMPUTER-LESBARE FASSUNG:
      (A) DATENTRAEGER: Tape
      (B) COMPUTER: IBM PC compatible
      (C) BETRIEBSSYSTEM: PC-DOS/MS-DOS
      (D) SOFTWARE: Patentln Release #1.0, Version #1.30 (EPA)
(2) ANGABEN ZU SEQ ID NO: 1:
   (i) SEQUENZKENNZEICHEN:
      (A) LAENGE: 412 Aminos"uren
      (B) ART: Aminos"ure
      (C) STRANGFORM: Einzelstrang
      (D) TOPOLOGIE: linear
   (ii) ART DES MOLEKÜLS: Peptid
   (iii) HYPOTHETISCH: NEIN
   (v) ART DES FRAGMENTS: N-Terminus
   (vi) URSPRÜNGLICHE HERKUNFT:
      (A) ORGANISMUS: Rthrobacter
      (B) STAMM: aurescens
   (xi) SEQUENZBESCHREIBUNG: SEQ ID NO: 1:
   Met Thr Leu Gln Lys Ala Gln Ala Ala Arg Ile Glu Lys Glu Ile Arg
   1               5                   10                  15
   Glu Leu Ser Arg Phe Ser Ala Glu Gly Pro Gly Val Thr Arg Leu Thr
               20                  25                  30
   Tyr Thr Pro Glu His Ala Ala Ala Arg Glu Thr Leu Ile Ala Ala Met
           35                  40                  45
   Lys Ala Ala Ala Leu Ser Val Arg Glu Asp Ala Leu Gly Asn Ile Ile
       50                  55                  60
   Gly Arg Arg Glu Gly Thr Asp Pro Glu Leu Pro Ala Ile Ala Val Gly
   65                  70                  75                  80
   Ser His Phe Asp Ser Val Arg Asn Gly Gly Met Phe Asp Gly Thr Ala
                   85                  90                  95
   Gly Val Val Cys Ala Leu Glu Ala Ala Arg Val Met Leu Glu Asn Gly
               100                 105                 110
   Tyr Val Asn Arg His Pro Phe Glu Phe Ile Ala Ile Val Glu Glu Glu
           115                 120                 125
   Gly Ala Arg Phe Ser Ser Gly Met Leu Gly Gly Arg Ala Ile Ala Gly
       130                 135                 140
   Leu Val Ala Asp Arg Glu Leu Asp Ser Leu Val Asp Glu Asp Gly Val
   145                 150                 155                 160
   Ser Val Arg Gln Ala Ala Thr Ala Phe Gly Leu Lys Pro Gly Glu Leu
                   165                 170                 175
   Gln Ala Ala Ala Arg Ser Ala Ala Asp Leu Arg Ala Phe Ile Glu Leu
               180                 185                 190
   His Ile Glu Gln Gly Pro Ile Leu Glu Gln Glu Gln Ile Glu Ile Gly
           195                 200                 205
   Val Val Thr Ser Ile Val Gly Val Arg Ala Leu Arg Val Ala Val Lys
       210                 215                 220
   Gly Arg Ser Asp His Ala Gly Thr Thr Pro Met His Leu Arg Gln Asp
   225                 230                 235                 240
   Ala Leu Val Pro Ala Ala Leu Met Val Arg Glu Val Asn Arg Phe Val
                   245                 250                 255
   Asn Glu Ile Ala Asp Gly Thr Val Ala Thr Val Gly His Leu Thr Val
               260                 265                 270
   Ala Pro Gly Gly Gly Asn Gln Val Pro Gly Glu Val Asp Phe Thr Leu
           275                 280                 285
   Asp Leu Arg Ser Pro His Glu Glu Ser Leu Arg Val Leu Ile Asp Arg
       290                 295                 300
   Ile Ser Val Met Val Gly Glu Val Ala Ser Gln Ala Gly Val Ala Ala
   305                 310                 315                 320
   Asp Val Asp Glu Phe Phe Asn Leu Ser Pro Val Gln Leu Ala Pro Thr
   325 330 335
   Met Val Asp Ala Val Arg Glu Ala Ala Ser Ala Leu Gln Phe Thr His
   340 345 350
   Arg Asp Ile Ser Ser Gly Ala Gly His Asp Ser Met Phe Ile Ala Gln
   355 360 365
   Val Thr Asp Val Gly Met Val Phe Val Pro Ser Arg Ala Gly Arg Ser
   370 375 380
   His Val Pro Glu Glu Trp Thr Asp Phe Asp Asp Leu Arg Lys Gly Thr
   385 390 395 400
   Glu Val Val Leu Arg Val Met Lys Ala Leu Asp Arg
   405 410